# EUROPEAN PATENT SPECIFICATION

(11) **EP 1 037 602 B1**
(45) Date of publication and mention of the grant of the patent: **15.10.2003**
(21) Application number: 98966318.2
(22) Date of filing: 10.12.1998
(51) Int. Cl.: A61K 7/50, A61K 7/00

(54) **MOUSSE-FORMING SHAMPOO COMPOSITIONS**
SCHAUMFORMENDE SHAMPOOZUSAMMENSETZUNGEN
COMPOSITIONS DE SHAMPOOING MOUSSANT

(30) Priority: 19.12.1997 GB 9726969
(43) Date of publication of application: 27.09.2000
(73) Proprietor: UNILEVER PLC, London EC4P 4BQ (GB); UNILEVER N.V., 3133 AT Vlaardingen (NL)
(72) Inventor: COUPE, Serge, Aime, Patrick, c/o Elida Faberge, 60881 Le Meux Cedex 139 (FR); REID, Euan, Stuart, Unilever Res. Port Sunlight, Wirral, Merseyside L63 3JW (GB); STEER, David, Charles, Unilever Res. Port Sunlight, Wirral, Merseyside L63 3JW (GB)
(74) Representative: Griffiths, Helen Sarah
(86) International application number: EP9808149
(87) International publication number: WO99032070

(56) References cited:
- EP-A- 0 567 326
- EP-A- 0 674 898
- WO-A-96/03969
- WO-A-96/31188
- GB-A- 2 190 393

## Description

### FIELD OF THE INVENTION

The present invention relates to mousse-forming shampoo compositions. More particularly, the invention relates to mousse-forming shampoo compositions which include emulsified particles of a conditioning agent such as a silicone and which impart good conditioning benefits to hair and/or skin.

### BACKGROUND AND PRIOR ART

Mousses are a particularly convenient and pleasant-to-use product form for hair treatment formulations. The product is generally applied to the user's hand, where it forms a creamy foam which can be easily worked through the hair.

Such mousses have found widespread use in the context of hair styling products. The conventional hair styling mousse generally utilises a water soluble hair styling polymer, water, possibly a conditioning agent, an emulsifier, aesthetic agents and an aerosol propellant. The mousse is typically applied to hair dampened with water, spread through the hair and allowed to dry, giving a temporary set which can be removed by water or by shampooing.

It would be desirable to provide a rinse-off surfactant-based cleansing shampoo in a mousse product form. Consumers appreciate the ease of dispensing and application of a mousse, and the way it can be worked through the hair without getting into the eyes. The latter would be particularly advantageous in the context of formulations based primarily on cleansing surfactants which can sometimes be harsh and irritating to the eyes.
However, prior art systems of this type have not achieved much success, largely because the level of conditioning they deliver is insufficient for many people.

The problem stems mainly from the fact that the shampoo in the dispenser must dispense easily. This requirement is generally incompatible with shampoos which incorporate conditioning agents.

For example, silicones are highly desirable conditioning agents for incorporation into shampoos, as is well documented in the literature. However, the problem arises that the usual viscosity level required of the shampoo base in order to prevent the silicone from separating in the formulation is generally too high for effective dispensing of the shampoo from an aerosol formulation. This is manifest as a dispensing problem - the product will tend to dispense slowly and unevenly.

We have now found that rinse-off surfactant-based cleansing shampoo compositions can be formulated which deliver excellent conditioning performance from a mousse product form.

US 5,085,857 describes a hair shampoo incorporating a pre-formed aqueous emulsion of a silicone oil, in which the silicone is incorporated with a small particle size, less than 2 microns. The shampoos preferably contain a shear thinning polymer or crystalline suspending agent to enhance stability.

EP A 0 529 883 and EP A 0 674 898 disclose hair shampoos comprising a microemulsified silicone oil in combination with a cationic deposition polymer. The shampoo compositions are stated to have good mechanical stability, high optical transparency or translucency and excellent conditioning ability.

None of these publications disclose or suggest that an emulsified conditioning agent such as a silicone can be utilised in a propellant-driven system such as a mousse-forming product.

### SUMMARY OF THE INVENTION

The present invention provides a mousse-forming cleansing shampoo composition having improved conditioning performance comprising:
(A) a foamable concentrate comprising:
   (i) at least one surfactant;
   (ii) emulsified particles of a conditioning agent having a particle size of ≤ 1 micron;
   (iii) a deposition polymer for the emulsified particles;
   (iv) an aqueous carrier; and
(B) an aerosol propellant.

### DETAILED DESCRIPTION AND PREFERRED EMBODIMENTS

### Foamable Concentrate

The mousse-forming cleansing shampoo composition of the invention comprises a foamable concentrate and an aerosol propellant. The term "concentrate" will be used to refer to the liquid component of the shampoo composition other than the propellant. The term "mousse", as used herein, is the same as foam, and refers to the dispensed product unless otherwise specified.

In general, for optimum dispensability of the product, we have found that the viscosity of the foamable concentrate should not exceed 3000 10⁻³ Pas (cps).

The viscosity of the foamable concentrate suitably ranges from 1 to 3000, preferably from 10 to 2000, ideally from 100 to 1000 10⁻³ Pas (cps).

Viscosity is measured in the conventional manner using a rotary viscometer (Brookfield Viscometer, LVT type, Rotor No.3, 12 rpm after 30 sec. at 25 degrees C).

In order to achieve such suitable viscosities as described above for the foamable concentrate, it is particularly preferred that the foamable concentrate be substantially free of crystalline suspending agents. By "substantially free" it is generally meant that the level of such agents be about 0.5% or less, preferably about 0.1% or less, ideally no more than about 0.05% by weight of the foamable concentrate.

Crystalline suspending agents include long chain (e.g. C8-C22) acyl derivative materials and long chain amine oxides, such as ethylene glycol long chain esters, alkanolamides of long chain fatty acids, long chain esters of long chain fatty acids, glyceryl long chain esters, long chain esters of long chain alkanolamides, and long chain alkyl dimethyl amine oxides. Common suspending agents of this type are ethylene glycol esters of C14-C22 fatty acids (e.g. ethylene glycol distearate), C16-C22 fatty acid alkanolamides (e.g. stearic monoethanolamide, stearic monoisopropanolamide), C16-C22 alkyl dimethyl amine oxides and N,N-dihydrocarbyl (C12-C22) amidobenzoic acid and salts thereof.

It may in some cases also be preferable, in order to achieve suitable viscosities as described above for the foamable concentrate, to incorporate therein a rheology modifier such as a thinner. Suitable thinners include polyethylene glycol (PEG), polypropylene glycol (PPG), sodium xylene sulphonate, sodium toluene sulphonate and urea. Preferred thinners are PEG 400 and PPG 400.

### Conditioning Agent

The foamable concentrate comprises emulsified particles of a conditioning agent having a particle size of ≤ 1 micron.

It is particularly preferred that the conditioning agent is in the form of microemulsified particles. Typically such microemulsified particles will have a particle size of ≤ 0.15 microns, suitably from 0.01 to 0.15 microns.

Particle size may be measured by means of a laser light scattering technique, using a 2600D Particle Sizer from Malvern Instruments.

As used herein, the term "conditioning agent" includes any material which is used to give a particular conditioning benefit to hair and/or skin. For example, in shampoo compositions for use on the skin, materials such as moisturisers, essential oils, sun-protective or after-sun treatment materials, or occlusive oils may be used. In shampoo compositions for use on the hair, suitable materials are those which deliver one or more benefits relating to shine, softness, combability, wet-handling, anti-static properties, protection against damage, body, volume, stylability and manageability.

Preferred conditioning agents for use in the present invention include silicones. Suitable silicones may be one or more polyalkyl siloxanes, one or more polyalkylaryl siloxanes, or mixtures thereof. The silicone is insoluble in the aqueous matrix of the composition and so is present as dispersed particles, in an emulsified form.

The viscosity of the silicone itself (not the emulsion or the final shampoo composition) preferably ranges from 10,000.10⁻³ Pas (cps) to 5 million 10⁻³ Pas (cps).

Suitable polyalkyl siloxanes include polydimethyl siloxanes which have the CTFA designation dimethicone, having a viscosity of up to 100,000 10⁻⁶ m²/s (centistokes) at 25 degrees C. These siloxanes are available commercially from the General Electric Company as the Viscasil series and from Dow Corning as the DC 200 series. The viscosity can be measured by means of a glass capillary viscometer as set out further in Dow Corning Corporate Test Method CTM004 July 20 1970.

Also suitable is polydiethyl siloxane.

Also suitable are silicone gums, such as those described in US Pat. No. 4,152,416 (Spitzer), and on General Electric Silicone Rubber product Data Sheet SE 30, SE 33, SE 54 and SE 76. "Silicone gum" denotes polydiorganosiloxanes having a molecular weight of from 200,000 to 1,000,000 and specific examples include polydimethyl siloxane polymers, polydimethyl siloxane/diphenyl/methylvinylsiloxane copolymers, polydimethylsiloxane/methylvinylsiloxane copolymers and mixtures thereof.

Aminofunctional silicones which have the CTFA designation amodimethicone, are also suitable for use in the compositions of the invention, as are polydimethyl siloxanes having hydroxyl end groups (which have the CTFA designation dimethiconol).

Various methods of making microemulsions of particles of silicones for use in the invention are available and are well known and documented in the art.

One particularly preferred technique for making silicone microemulsions is that described in EP-A-228575.

In that document there is described a method of making a stable microemulsion of high molecular weight silicone polymer and water by sequentially adding at an effective rate a standard emulsion comprising polydiorganosiloxane precursor, surfactant and water to a polymerization catalyst medium while mixing to form a clear, stable aqueous microemulsion of polydiorganosiloxane.

Another method of making suitable microemulsions for use in the invention are described in EP-A-0 138 192.

Alternatively, suitable microemulsions for use in the invention are commercially available in a pre-microemulsified form. This is particularly preferred since the pre-formed microemulsion can be incorporated into the foamable concentrate by simple mixing. Examples of suitable pre-formed microemulsions include microemulsions DC2-1865, DC2-1870, and DC2-1391, all available from Dow Corning. These are all microemulsions of dimethiconol.

The amount of silicone incorporated into the shampoo compositions of the invention depends on the level of conditioning desired and the material used. A preferred amount is from 0.01 to about 10% by weight of the foamable concentrate although these limits are not absolute. The lower limit is determined by the minimum level to achieve conditioning and the upper limit by the maximum level to avoid making the hair and/or skin unacceptably greasy. We have found that an amount of silicone of from 0.5 to 1.5% by weight of the foamable concentrate, is a particularly suitable level.

A further preferred class of conditioning agents are hair body and volume enhancing materials. Examples are cross-linked silicone gums and per-alk(en)yl hydrocarbon materials.

Suitable cross-linked silicone gums are described in WO 96/31188. A preferred example is the material available from Dow Corning as DC X2-1787.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of hair body and volume enhancing material incorporated into the shampoo compositions of the invention depends on the level of enhancement desired and the material used. A preferred amount is from 0.01 to about 10% by weight of the foamable concentrate although these limits are not absolute. The lower limit is determined by the minimum level to achieve the body and volume enhancing effect and the upper limit by the maximum level to avoid making the hair unacceptably stiff. We have found that an amount of hair body and volume enhancing material of from 0.1 to 2% by weight of the foamable concentrate, is a particularly suitable level.

### Surfactant

The foamable concentrate comprises one or more surfactants, to provide a cleansing benefit. Surfactant will also be present as emulsifier for the microemulsified particles of silicone.

Further surfactant(s) will be present as an additional cleansing ingredient if sufficient for cleansing purposes is not provided as the emulsifier for the microemulsified particles of silicone. This further cleansing surfactant may be the same surfactant as the emulsifier, or may be different.

Suitable emulsifiers are well known in the art and include anionic and nonionic surfactants. Examples of anionic surfactants used as emulsifiers for the silicone particles are alkylarylsulphonates, e.g., sodium dodecylbenzene sulphonate, alkyl sulphates e.g., sodium lauryl sulphate, alkyl ether sulphates, e.g., sodium lauryl ether sulphate nEO, where n is from 1 to 20 alkylphenol ether sulphates, e.g., octylphenol ether sulphate nEO where n is from 1 to 20, and sulphosuccinates, e.g., sodium dioctylsulphosuccinate.

Examples of nonionic surfactants used as emulsifiers for the silicone particles are alkylphenol ethoxylates, e.g., nonylphenol ethoxylate nEO, where n is from 1 to 50, alcohol ethoxylates, e.g., lauryl alcohol nEO, where n is from 1 to 50, ester ethoxylates, e.g., polyoxyethylene monostearate where the number of oxyethylene units is from 1 to 30.

Cleansing surfactants are typically selected from anionic, nonionic, amphoteric and zwitterionic surfactants, and mixtures thereof.

Suitable anionic cleansing surfactants for shampoo compositions of the invention include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, alpha-olefin sulphonates and acyl methyl taurates, especially their sodium, magnesium ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionics include sodium lauryl sulphate, sodium lauryl ether sulphate, ammonium lauryl sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauroyl isethionate, and sodium N-lauryl sarcosinate.

Nonionic cleansing surfactants suitable for use in shampoo compositions of the invention may include condensation products of aliphatic (C₈-C₁₈) primary or secondary linear or branched chain alcohols or phenols with alkylene oxides, usually ethylene oxide and generally having from 6 to 30 ethylene oxide groups. Other suitable nonionics include alkylpolyglycosides and mono- or di-alkyl alkanolamides. Examples of the latter nonionics include coco mono- or diethanolamide and coco mono-isopropanolamide.

Amphoteric and zwitterionic cleansing surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates and alkyl amidopropyl hydroxysultaines. Examples include lauryl amine oxide, cocodimethyl sulphopropyl betaine and preferably lauryl betaine, cocamidopropyl betaine and sodium cocamphopropionate.

The total amount of surfactant (including that used as emulsifier for the microemulsified particles of high viscosity silicone) is generally from 3 to 50% by weight, preferably from 5 to 30%, more preferably from 10% to 25% by weight of the foamable concentrate.

### Deposition polymer

The foamable concentrate contains a deposition polymer for the microemulsified particles of silicone. By "deposition polymer" is meant an agent which enhances deposition of the particles of silicone from the shampoo composition of the invention onto the intended site during use, i.e. the hair and/or the scalp.

The deposition polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic charge density of the deposition polymer, which is defined as the reciprocal of the molecular weight of a monomeric unit of the polymer containing one charge, should typically be at least 0.1 meq/g, preferably above 0.8 or higher. The cationic charge density should typically not exceed 4 meq/g. It is preferably less than 3 and more preferably less than 2 meq/g. The charge density can be measured using conductimetric analysis and should be within the above limits at the desired pH of use, which will in general be from about 3 to 9 and preferably between 4 and 8.

The cationic nitrogen-containing group will generally be present as a substituent on a fraction of the total monomer units of the deposition polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

Suitable cationic amino and quaternary ammonium monomers include, for example, vinyl compounds substituted with dialkyl aminoalkyl acrylate, dialkylamino alkylmethacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidine, e.g., alkyl vinyl imidazolium, alkyl vinyl pyridinium, and alkyl vinyl pyrrolidine salts. The alkyl portions of these ,monomers are preferably lower alkyls such as the C1-C3 alkyls, more preferably C1 and C2 alkyls.

Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C1-C7 hydrocarbyls, more preferably C1-C3, alkyls.

The deposition polymer can comprise mixtures of monomer units derived from amine- and/or quaternary ammonium-substituted monomer and/or compatible spacer monomers.

Suitable deposition polymers include, for example: cationic copolymers of 1-vinyl-2-pyrrolidine and 1-vinyl-3-methylimidazolium salt (e.g., Chloride salt) (referred to in the industry by the Cosmetic, Toiletry, and Fragrance Association, "CTFA". as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in UK Application No. 9403156.4.

Other cationic deposition polymers that can be used include cationic guar gum derivatives, such as guar hydroxypropyltrimonium chloride (Commercially available from Celanese Corp. in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the deposition polymer is selected from cationic polyacrylamides and cationic guar derivatives. Particularly preferred deposition polymers are JAGUAR C13S with a cationic charge density of 0.8meq/g. Other particularly suitable materials include JAGUAR C15, JAGUAR C17 and JAGUAR C16 and JAGUAR C162.

The deposition polymer may be present in an amount of from about 0.01 to about 10% by weight of the total composition, preferably from about 0.01 to about 1% by weight, more preferably from about 0.04 to about 0.5% by weight of the foamable concentrate.

### Aqueous carrier

The foamable concentrate comprises an aqueous carrier, water forming the basis of the continuous phase of the microemulsion of particles of silicone conditioning agent. Water is generally present in an amount of from about 20 to about 99% by weight of the foamable concentrate.

### Propellant

Shampoo compositions of the invention contain an aerosol propellant (B). This agent is responsible for expelling the other materials from the container and forming the mousse character.

The propellant gas can be any liquefiable gas conventionally used for aerosol containers. Examples of suitable propellants include dimethyl ether, propane, n-butane and isobutane, used singly or admixed. Other examples of propellants are nitrogen, carbon dioxide, compressed air and fluorohydrocarbons such as the material sold by Du Pont under the trade name DYMEL 152a.

The amount of the propellant gases is governed by normal factors well known in the aerosol art. For mousses the level of propellant is generally from about 3 to about 15%, optimally from about 4 to about 10% for creamy foam and good sensory feel.

### Optional Ingredients

Compositions of this invention may contain any other ingredient normally used in hair treatment formulations. These other ingredients may include hair styling resins, colouring agents, antifoam agents, proteins, moisturising agents, antioxidants, fragrances, antimicrobials and sunscreens. Each of these ingredients will be present in an amount effective to accomplish its purpose.

### Packaging

Compositions of the invention are typically prepared by charging a suitable pressurisable container with the foamable concentrate, then sealing the container and charging it with propellant (B) according to conventional techniques.

The invention will now be illustrated by the following nonlimiting examples.

All parts, percentages and proportions referred to are by weight unless otherwise indicated.

### EXAMPLES

The following Examples illustrate mousse-forming shampoo compositions according to the present invention.

### Example 1

A foamable concentrate was made up having the following formulation:

| **wt%** | **Ingredient** | **Chemical Name** |
|---|---|---|
| 9.0% | SLES 2EO | Sodium lauryl ether sulphate (2EO) |
| 4.0% | CAPB | Cocamidopropylbetaine |
| 4.0% | Laureth-7 | Polyethylene glycol (7) ether of lauryl alcohol |
| 3.2% | DC2-1391 | Cross-linked dimethiconol (25% microemulsion, ex Dow Corning) |
| 2.0% | PPG 400 | Polyoxypropylene (9) |
| 0.5% | Sodium benzoate | |
| 0.5% | Fragrance | |
| 0.3% | Polymer JR400 | Polyquaternium-10 |
| to 100% | deionised water | |

The foamable concentrate was formulated into an aerosol mousse using 93 parts of the foamable concentrate and 7 parts CAP 40 (butane, 2.7 bar).

### Example 2

A foamable concentrate was made up having the following formulation:

| **wt%** | **Ingredient** | **Chemical Name** |
|---|---|---|
| 14.0% | SLES 2EO | Sodium lauryl ether sulphate (2EO) |
| 3.0% | DCX2-1787 | Emulsion polymerised dimethiconol containing 0.6% cross-linking, (55% aqueous emulsion, ex Dow Corning) |
| 2.0% | CAPB | Cocamidopropylbetaine |
| 2.0% | PPG 400 | Polyoxypropylene (9) |
| 0.5% | Sodium benzoate | |
| 0.5% | Fragrance | |
| 0.05% | JAGUAR C13S | Guar hydroxypropyltrimonium chloride |
| to 100% | deionised water | |

The foamable concentrate was formulated into an aerosol mousse using 93 parts of the foamable concentrate and 7 parts CAP 40 (butane, 2.7 bar).

## Claims

1. A mousse-forming cleansing shampoo composition having improved conditioning performance comprising:
(A) a foamable concentrate comprising:
(i) at least one surfactant;
(ii) emulsified particles of a conditioning agent having a particle size of ≤ 1 micron;
(iii) a deposition polymer for the emulsified particles;
(iv) an aqueous carrier; and
(B) an aerosol propellant.

2. A composition according to claim 1, in which the foamable concentrate is substantially free of crystalline suspending agents.

3. A composition according to claim 1 or claim 2, in which the foamable concentrate further comprises a rheology modifier selected from the group consisting of polyethylene glycol (PEG), polypropylene glycol (PPG), sodium xylene sulphonate, sodium toluene sulphonate and urea.

4. A composition according to any preceding claim, in which the conditioning agent is pre-microemulsified dimethiconol.

5. A composition according to any of claims 1 to 3, in which the conditioning agent is a cross-linked silicone gum or a per-alk(en)yl hydrocarbon material.

6. A composition according to any preceding claim, in which the total amount of surfactant, including that used as emulsifier for the microemulsified particles of high viscosity silicone, is from 10% to 25% by weight of the foamable concentrate.

7. A composition according to any preceding claim, in which the deposition polymer is selected from the group consisting of cationic polyacrylamides and cationic guar derivatives.

8. A composition according to any preceding claim, in which the propellant gas is selected from the group consisting of dimethyl ether, propane, n-butane, isobutane and mixtures thereof.

## Patentansprüche

1. Schaumbildende reinigende Shampoozusammensetzung mit verbesserter konditionierender Leistung, umfassend:
(A) ein schäumbares Konzentrat, umfassend:
(i) mindestens ein Tensid;
(ii) emulgierte Teilchen eines konditionierenden Mittels mit einer Teilchengröße von ≤ 1 µm;
(iii) ein Abscheidungspolymer für die emulgierten Teilchen;
(iv) einen wässrigen Träger und
(B) ein Aerosoltreibmittel

2. Zusammensetzung nach Anspruch 1, worin das schäumbare Konzentrat im Wesentlichen frei von kristallinen suspendierenden Mitteln ist.

3. Zusammensetzung nach Anspruch 1 oder Anspruch 2, worin das schäumbare Konzentrat weiterhin ein Rheologiemodifizierungsmittel, ausgewählt aus der Gruppe, bestehend aus Polyethylenglycol (PEG), Polypropylenglycol (PPG), Natriumxylolsulfonat, Natriumtoluolsulfonat und Harnstoff, umfasst.

4. Zusammensetzung nach einem vorangehenden Anspruch, worin das Konditionierungsmittel vormikroemulgiertes Dimethiconol ist.

5. Zusammensetzung nach einem der Ansprüche 1 bis 3, worin das Konditionierungsmittel ein vernetztes Silikongummi oder ein Peralk(en)ylkohlenwasserstoffmaterial darstellt.

6. Zusammensetzung nach einem vorangehenden Anspruch, worin die Gesamtmenge an Tensid, einschließlich jenem, das als Emulgator für die mikroemulgierten Teilchen von hochviskosem Silikon verwendet wird, 10% bis 25 Gewichtsprozent des schäumbaren Konzentrats ist.

7. Zusammensetzung nach einem vorangehenden Anspruch, worin das Abscheidungspolymer aus der Gruppe, bestehend aus kationischen Polyacrylamiden und kationischen Guarderivaten, ausgewählt ist.

8. Zusammensetzung nach einem vorangehenden Anspruch, worin das Treibmittelgas aus der Gruppe, bestehend aus Dimethylether, Propan, n-Butan, Isobutan und Gemischen davon, ausgewählt ist.

## Revendications

1. Composition de shampoing moussant nettoyant ayant des propriétés de conditionnement améliorées, comprenant :
(A) un concentré moussant comprenant :
(i) au moins un tensioactif ;
(ii) des particules émulsionnées d'un agent de conditionnement ayant une taille de particules de ≥ 1 micron ;
(iii) un polymère de déposition pour les particules émulsionnées ;
(iv) un matériau aqueux formant support ; et
(B) un propulseur aérosol.

2. Composition selon la revendication 1, dans laquelle le concentré moussant ne contient substantiellement pas d'agents de mise en suspension cristallins.

3. Composition selon là revendication 1 ou la revendication 2, dans laquelle le concentré moussant comprend en outre un modificateur de rhéologie sélectionné à partir du groupe constitué du polyéthylène glycol (PEG), du polypropylène glycol (PPG), du xylène sulfonate de sodium, du toluène sulfonate de sodium et de l'urée.

4. Composition selon l'une quelconque des revendications précédentes, dans laquelle l'agent de conditionnement est du diméthiconol pré émulsionné.

5. Composition selon l'une quelconque des revendications 1 à 3, dans laquelle l'agent de conditionnement est une gomme de silicone réticulée ou un matériau hydrocarbure de per-alk (én) yle.

6. Composition selon l'une quelconque des revendications précédentes, dans laquelle la quantité totale de tensioactif, incluant celle utilisée en tant qu'émulsionnant pour les particules micro émulsionnées, est de 10 % à 25 % en poids du concentré moussant.

7. Composition selon l'une quelconque des revendications précédentes, dans laquelle le polymère de déposition est sectionné à partir du groupe constitué des polyacrylamides cationiques et des dérivés cationiques de guar.

8. Composition selon l'une quelconque des revendications précédentes, dans laquelle le gaz propulseur est sélectionné à partir du groupe constitué de l'éther de diméthyle, du propane, du n - butane, de l'isobutane et des mélanges de ceux-ci.
